Europäisches Patentamt

European Patent Office

Office européen des brevets

(19)

(11) Veröffentlichungsnummer : **0 016 387**
**B1**

## (12) EUROPÄISCHE PATENTSCHRIFT

(45) Veröffentlichungstag der Patentschrift :
26.01.83

(21) Anmeldenummer : 80101187.5

(22) Anmeldetag : 08.03.80

(51) Int. Cl.³ : **G 01 N 33/52, G 01 N 31/22**

(54) **Diagnostisches Mittel zum Nachweis von Bestandteilen von Flüssigkeiten.**

(30) Priorität : 15.03.79 DE 2910134

(43) Veröffentlichungstag der Anmeldung :
01.10.80 Patentblatt 80/20

(45) Bekanntmachung des Hinweises auf die Patenterteilung : 26.01.83 Patentblatt 83/04

(84) Benannte Vertragsstaaten :
**AT BE CH DE FR GB IT LU NL SE**

(56) Entgegenhaltungen :
**DE A 1 598 153**
**DE A 2 603 004**
**FR A 2 191 734**
**GB A 1 316 671**
**US A 3 784 358**
**US A 3 844 865**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Vogel, Peter, Dr.rer.nat.**
**Schubertweg 5**
**D-6944 Hemsbach (DE)**
Erfinder : **Braun, Hans-Peter, Dr.rer.nat.**
**Auf der Au 3**
**D-6944 Hemsbach (DE)**
Erfinder : **Berger, Dieter, Dr.rer.nat.**
**Bensheimer-Strasse 45**
**D-6806 Viernheim (DE)**
Erfinder : **Werner, Wolfgang, Dr.rer.nat.**
**Meissener Weg 39**
**D-6800 Mannheim 42 (DE)**

# 0 016 387

## Diagnostisches Mittel zum Nachweis von Bestandteilen von Flüssigkeiten

Die Erfindung bezieht sich auf ein diagnostisches Mittel der im Oberbegriff des Anspruchs 1 genannten Art. Der Nachweis der Inhaltsstoffe von Flüssigkeiten mittels Teststreifen gewinnt eine immer größere Bedeutung. Sie liefern in vielen Fällen einfache, preiswerte und schnelle Nachweisverfahren. Weitverbreitet sind Teststreifen in der Harndiagnostik als qualitative und semiquantitative Nachweisverfahren. Spezielle Teststreifen können auch zum Nachweis von Inhaltsstoffen von Blut und Serum in der ärztlichen Diagnose eingesetzt werden. Darüber hinaus werden Teststreifen auch zur Untersuchung von Getränken, Trinkwasser, Abwasser und anderen in der Industrie anfallenden Flüssigkeiten vielfach genutzt.

Eine Schwierigkeit für die quantitative Auswertung der Nachweis-Methoden insbesondere für hochmolekulare und korpuskuläre Bestandteile von Flüssigkeiten auf Teststreifen-Basis bestand bisher darin, daß fast ausschließlich Papier als saugender Träger eingesetzt wurde. Beispielsweise sei die Bestimmungsmethode vom Hämoglobin nach Tallqvist im Blut angeführt. Die Homogenität im Bezug auf Schichtdicke, Struktur und Zusammensetzung, wie sie für quantitative Teste erforderlich ist, ist bei Papieren nur schwer zu erreichen. Bei Verwendung von Papieren für quantitative Teste erweist es sich weiter als nachteilig, daß häufig eine exakte Dosierung des Probenmaterials notwendig ist.

Einen großen Fortschritt für die quantitative Bestimmung niedermolekularer Stoffe erbrachte die Verwendung von Filmen gemäß DE-C-1 598 153. Diese Filme können in ihren Eigenschaften den jeweiligen Analysenverfahren angepaßt werden. Sie verlangen keine präzise Dosierung des Untersuchungsguts und reagieren gleichermaßen mit Harn, Plasma, Serum oder Vollblut. Nach einer kurzen Einwirkzeit wird das überschüssige Probenmaterial einfach abgewischt. Auf Grund der geringen Porengröße dieser Filme ermöglichen sie eine Abtrennung der dispergierten oder suspendierten Bestandteile der zu untersuchenden Lösung, bspw. der Erythrozyten des Blutes, von den niedermolekularen, gelösten Inhaltsstoffen.

Diese Filme können, außer den für die Nachweisreaktion erforderlichen Reagenzien, auch übliche Füllstoffe und Hilfsstoffe wie Kreide, Titandioxid etc. oder Pigmente zur Erhöhung der Remission enthalten. Die in den verschiedenen Ausführungsbeispielen angegebenen Mengen dieser Stoffe reichen aber nicht aus, um die Durchlässigkeit der Filmoberfläche zu verändern. Diese Filme können aber nicht eingesetzt werden, wenn celluläre Bestandteile oder große Moleküle mit Molgewichten über 50.000, z.B. Enzyme, bestimmt werden sollen. Derartige Substanzen dringen nicht oder nicht ausreichend in den Film ein, um eine meßbare Reaktion zu bewirken. Aus diesem Grunde lassen sich mit diesem Filmprinzip z.B. keine brauchbaren Teste für den Nachweis von Hämoglobin, Cholesterin in Lipoproteinen oder Enzymen herstellen.

In der GB-A-13 16 671 sind poröse Blätter oder Filme beschrieben, die dadurch erhalten werden, daß man einer Lösung des Kunststoffs 50 % eines Füllstoffs und entweder eine größere Menge eines Lösungsmittels zusetzt, so daß beim Verdampfen desselben Hohlräume entstehen, oder die erhaltene Folie mechanisch verstreckt, so daß sie entlang der Füllstoff/Kunststoffgrenzen aufreißt und ebenfalls Hohlräume gebildet werden. Die erhaltenen Folien sollen als Papier oder Lederersatz verwendet werden und eine möglichst große Luftdurchlässigkeit aufweisen.

In der DE-A-26 03 004 sind streifenförmige Diagnoseeinrichtungen beschrieben, die entweder aus Filterpapier oder aus einem mikroporösen, Kapillarität aufweisenden Kunststoffblatt aus Polyvinylchlorid, das mit 50 % Siliziumdioxid versetzt ist, bestehen. Die inneren Oberflächen dieser Träger sind mit einem Reagenz belegt, welches imstande ist, das Substrat zu immobilisieren. Für den Test würde das Substrat durch den Streifen chromatographiert und die Kapillarwanderungsstrecke mit einer Indikatorlösung sichtbar gemacht. Da die Reagenzien nicht in den Träger eingebettet sind, sondern nur auf die Oberfläche aufgebracht sind, müssen sie durch eine kovalente chemische Bindung fixiert werden, was die Anwendbarkeit der Methoden auf wenige spezielle Fälle einschränkt.

Es wurde nun völlig überraschend gefunden, daß saugende, « offene » Filme erhalten werden, wenn in die wässrige Dispersion von filmbildenden organischen Kunststoffen, aus der der unlösliche Film hergestellt wird, Feststoffe in Form feiner, unlöslicher, organischer oder anorganischer Partikel mit einer Größe von 0,2-20 µm gegeben werden. Filmbildende Substanz und zugesetzter Füllstoff müssen in der zu untersuchenden Flüssigkeit, üblicherweise einer wässrigen Lösung, unlöslich sein. Da der Füllstoff selbst nicht « reagiert », ist seine Zusammensetzung unkritisch. Verwendet werden kann beispielsweise Cellulose, Kieselgur, Kieselgel, gefällter Gips, Calciumcarbonat, Kaolin, Polyamid, Glas u.a. Das Gewichtsverhältnis der Menge dieser Stoffe, im folgenden « Füllstoffe » genannt, zur Menge des filmbildenken Kunststoffs soll 20 : 1 bis 2 : 1 bevorzugt 5 : 1 bis 2 : 1 betragen. Das Mengenverhältnis hängt von dem vorgesehenen Verwendungszweck ab. Mit steigenden Mengen Füllstoff und steigender spezifischer Oberfläche des verwendeten Materials wird der Film saugfähiger.

Überschreitet der Anteil an Füllstoff eine bestimmte Grenze, so wird der Film mechanisch instabil. Wird zu wenig Füllstoff in den Film gegeben, wird er für hochmolekulare oder korpuskuläre Bestandteile durchlässig.

Unter hochmolekularen Inhaltsstoffen werden solche mit einem Mol-Gewicht von über 50.000 verstanden.

Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, z.B. von Butadien und Styrol oder von Maleinsäureester und Vinylacetat, jedoch können auch andere filmbildende, natürliche und synthetische organische Polymere sowie Mischungen derselben in Form von wässrigen Dispersionen, verwendet werden.

Die Dispersionen lassen sich auf einer Unterlage zu einer gleichmäßigen Schicht verstreichen, die nach dem Trocknen einen wasserfesten Film ergibt. Die trockenen Filme haben eine Dicke von 10 μm bis 500 μm vorzugsweise von 30-200 μm.

Ein besonderer Vorteil gegenüber saugfähigen Papieren besteht darin, daß solche Filme einfacher, gleichmäßiger und reproduzierbarer gefertigt werden können. Der Film kann mit der Unterlage als Träger zusammen verwendet werden oder für die Nachweisreaktion von ihr abgezogen und bspw. auf einen anderen Träger aufgebracht werden. Träger für die Filme sind vorzugsweise Kunststoff-Folien. Es können aber auch andere Folien, Papiere, Kunststoff-Tafeln, Glas, Metall usw. als Träger eingesetzt werden, wenn es der Verwendungszweck verlangt.

Die für die Nachweisreaktion erforderlichen Reagenzien werden normalerweise direkt in die Dispersion gegeben. Sofern vorteilhaft, kann der gebildete Film aber mit ihnen auch imprägniert werden. Auch eine Vorimprägnierung der Füllstoffe mit den Reagenzien ist möglich. Die Verfahren lassen sich auch derart kombinieren, daß z.B. bestimmte Bestandteile in die Dispersion gegeben werden und andere auf den Film nachimprägniert werden. Dadurch läßt sich eine gewisse, räumliche Trennung der Bestandteile erreichen, was zu stabileren oder reaktiveren Testen führen kann. Eine weitere Möglichkeit, Rezepturbestandteile voneinander zu trennen, besteht darin, sie auf verschiedene Beschichtungsmassen zu verteilen und diese in der optimalen Reihenfolge nacheinander zu beschichten, so daß ein Mehrschichten-System entsteht.

Sofern nötig, lassen sich Verdickungsmittel, Emulgatoren, Dispergiermittel, Pigmente, wie z.B. Titandioxid, Weichmacher, Netzmittel usw., zusetzen.

Dispergiermittel, Emulgatoren und Verdickungsmittel dienen zum Herstellen und Stabilisieren der Dispersionen. Pigmente, wie z.B. Titandioxid, die identisch mit dem Füllstoff sein können, verbessern die Remissionseigenschaften von Filmen, indem sie für möglichst geringe Transparenz und erhöhte Remission der Filme sorgen. Dies ist insbesondere dann von Vorteil, wenn die so erhaltenen diagnostischen Prüfmittel remissionsphotometrisch ausgewertet werden sollen. Mit Weichmachern lassen sich die Eigenschaften der Filmbeschichtungsmassen sowie der Filme optimieren. z.B. werden ihre Standfestigkeit, ihre Viskosität, ihre Haftung auf der zu beschichtenden Unterlage u.a. verbessert. Netzmittel werden eingesetzt, um eine bessere Benetzung des Films durch das Probengut zu erreichen. Sie können gleichzeitig auch Reaktionen katalysieren oder Rezepturen stabilisieren oder die Reaktionsfarbe brillanter und kontrastreicher machen.

Die hierin beschriebenen Filme werden vorzugsweise beim Nachweis der Inhaltsstoffe von Körperflüssigkeiten, wie Harn, Blut, Serum und Speichel eingesetzt, können jedoch in geeigneter Modifizierung auch in anderen wäßrigen Flüssigkeiten wie Trinkwasser, Abwasser etc. und gegebenenfalls auch in organischen Lösungsmitteln, in denen sie unlöslich sind, eingesetzt werden.

Gegenstand der vorliegenden Erfindung ist ein diagnostisches Mittel zum Nachweis von korpuskulären oder hochmolekularen Inhaltsstoffen von Flüssigkeiten, wie es in den Ansprüchen näher gekennzeichnet ist.

Die großen Vorteile bestehen darin, daß der Film, der die benötigten Reagenzien aufnimmt, auf den vorgesehenen Verwendungszweck gezielt eingestellt werden kann, indem man die besten geeigneten Filmbildner, Füllstoffe usw. auswählt. Ein weiterer großer Vorteil ergibt sich daraus, daß auf präzise Probendosierung verzichtet werden kann, weil der Film das Probenmaterial selbst dosiert. Der Überschuß wird einfach nach einer gewissen Einwirkungszeit abgewischt.

Im folgenden werden einige Beispiele für das erfindungsgemäße Mittel gegeben.

## Beispiel 1

Zur Herstellung eines Reagenzfilmes zum Nachweis von Cholesterin im Serum wird eine Dispersion folgender Zusammensetzung bereitet :

| | |
|---|---|
| Cellulose | 5 g |
| Polyvinylpropionat-Dispersion (50 % in Wasser) | 3 g |
| Methylhydroxypropyl-Cellulose | 0,042 g |
| Titandioxid-Pulver | 2 g |
| Cholesterinesterase | 1 200 U |
| Cholesterinoxidase | 800 U |
| Peroxidase | 26 000 U |
| Gallussäure | 0,003 2 g |
| Eine Lösung von 0,2 g 3,3',5,5'-Tetramethylbenzidin und 0,17 g Dioctylnatriumsulfosuccinat in 0,74 ml Aceton | 1 ml |
| Kaliumdihydrogen-Phosphat | 0,049 g |

| Dinatriumhydrogenphosphatdihydrat | 0,167 g |
| dest. Wasser | 19,5 ml |

Die Mischung wird auf eine Polycarbonat-Folie in einer 300 μm dicken Schicht aufgetragen und anschließend mit Warmluft getrocknet. Die so erhaltene Reagenzschicht ergab mit cholesterinhaltigen Seren in Abhängigkeit von der CH-Konzentration gut abgestufte, blaue Färbungen.

Nach Justieren des mit einer linearen Skala versehenen Remissionsphotometers (Reflomat ®) mit dem unbenutzten Teststreifen auf 0 Skalenteile und mit einer schwarzen Folie auf 100 Skalenteile erhält man für die den Blaufärbungen entsprechenden Cholesterinkonzentrationen die folgenden Meßwerte :

| 0 mg | 0 Skalenteile |
| 100 mg | 6 Skalenteile |
| 200 mg | 44 Skalenteile |
| 300 mg | 66 Skalenteile |
| 400 mg | 76 Skalenteile |
| 500 mg | 83 Skalenteile |
| 600 mg | 88 Skalenteile |
| Schwarze Kontrollfolie | 100 Skalenteile |

## Beispiel 2

Für einen Test zum Nachweis von Erythrozyten im Harn wurde zunächst ein Film aus folgender Mischung hergestellt :

| Polyvinylpropionat-Dispersion (50 % in Wasser) | 50 g |
| Kaolin | 60 g |
| Dioctylnatriumsulfosuccinat | 2 g |
| dest. Wasser | 140 ml |

Die Mischung wurde in einer 400 μm starken Schicht auf eine Polycarbonat-Folie aufgetragen und anschließend mit Warmluft getrocknet. Der so erhaltene Film wird mit den unten aufgeführten Lösungen I und II imprägniert und nach jeder Imprägnierung mit Warmluft getrocknet.

Lösung I

| EDTA · Na$_2$ | 1 g |
| Trinatriumcitrat · 2H$_2$O | 15,7 g |
| Citronensäure · H$_2$O | 3,48 g |
| Phosphorsäuretrimorpholid | 30,5 g |
| Benzo-lichtgelb | 0,048 g |
| dest. Wasser | 190 ml |

Dazu werden 3,2 g 2,5-Dimethyl-2,5-dihydroperoxihexan in 60 ml Methanol gelöst gegeben.

Lösung II

| 3,3',5,5'-Tetramethylbenzidin | 0,56 g |
| Dioctylnatriumsulfosuccinat | 1,0 g |
| Phenanthridin | 1,4 g |
| Phenylsemicarbazid | 0,06 g |
| Toluol | 100 ml |
| Methoxyethanol | 10 ml |
| Petrolether | 90 ml |

Der so erhaltene Film ergibt mit erythrozytenhaltigen Harnen grüne Färbungen.

## Beispiel 3

Für den Nachweis von Hämoglobin in Blut wurde ein Reagenzfilm aus folgender Mischung hergestellt :

| Cellulose | 20 g |
| Polyvinylpropionat-Dispersion (50 % in Wasser) | 15 g |
| Dioctylnatriumsulfosuccinat | 0,3 g |
| 0,5 mol PO$_4$-Puffer, PH = 7 | 75 ml |

Die Mischung wurde in einer 200 μm dicken Schicht auf eine Polyester-Folie aufgetragen und mit Warmluft getrocknet.

Läßt man aus einer Verdünnungsreihe von Blut mit verschiedenem Hämoglobin-Gehalt jeweils 1 Tropfen 1 Minute auf dem Test einwirken, so erhält man nach Abwischen des Tropfens mit Watte abgestufte Färbungen.

Vermißt man die entstandene Färbung der Teststreifen am Remissionsphotometer (PMQ3 von Zeiß) bei 540 nm, dann erhält man in Abhängigkeit von der Hämoglobinkonzentration folgende Meßwerte :

| g HB/l | % R |
| --- | --- |
| 53 | 46,1 |
| 100 | 38,8 |
| 150 | 31,7 |
| 201 | 25,5 |

## Beispiel 4

Zur Feststellung der Grenzen des Verfahrens wurde in einer zu Beispiel 3 analogen Mischung Filmöffner und Filmbildner variiert und Filme mit folgenden Eigenschaften erhalten :

| Filmöffner/ | | Filmbildner | Eigenschaften bei Reaktion mit den Erythrozyten im Blut |
| --- | --- | --- | --- |
| Kieselgur | : | Polyvinylpropionat | |
| 0,5 | : | 1 | Stabiler Film, Erythrozyten dringen nur an einigen Stellen ein. |
| 1 | : | 1 | Stabiler Film, gleichmäßige Reaktion des ganzen Films. |
| 4 | : | 1 | Gleichmäßige Reaktion des ganzen Films, Filmmaterial wird beim Abwischen bereits teilweise abgelöst. |
| Gips | : | Polyvinylpropionat | |
| 3 | : | 1 | Stabiler Film, geringfügige Anfärbung. |
| 4 bis 10 | : | 1 | Stabiler Film, gleichmäßige, tiefe Anfärbung. |
| 20 | : | 1 | Bei geringem Druck abwischfester Film, gleichmäßige Anfärbung, Tropfen « chromatographiert » am Rand. |

## Ansprüche

1. Diagnostiches Mittel zum Nachweis von Inhaltsstoffen von Flüssigkeiten bestehend aus einem flüssigkeitsbeständigen Film. der aus einer wäßrigen Dispersion von filmbildenden organischen Kunststoffen hergestellt ist und dem die für den Nachweis erforderlichen Reagenzien sowie Füllstoffe in Form unlöslicher anorganischer oder organischer Partikel einer Größe zwischen 0,2 und 20 Mikrometern beigemischt sind, dadurch gekennzeichnet, daß zur Öffnung des Films für Inhaltsstoffe eines Molekulargewichts von über 50.000 sowie für Inhaltsstoffe in Form von korpuskulären Bestandteilen das Gewichtsverhältnis von Füllstoffmenge zur Kunststoffmenge im Bereich von 2 : 1 bis 20 : 1 liegt.

2. Diagnostisches Mittel gemäß Anspruch 1, dadurch gekennzeichnet, daß der Film auf einem Träger befestigt ist.

## Claims

1. Diagnostic agent for the detection of component materials in liquids, consisting of a water- resistant film which is produced from an aqueous dispersion of film-forming organic synthetic resins and with which are admixed the reagents necessary for the detection, as well as filling materials in the form of insoluble inorganic or organic particles with a size of between 0.2 and 20 micrometers, characterised in that for the opening of the film for component materials with a molecular weight of over 50,000, as well as for component materials in the form of corpuscular components, the weight ratio of the amount of filling material to the amount of synthetic resin lies in the range of from 2 : 1 to 20 : 1.

2. Diagnostic agent according to claim 1, characterised in that the film is fixed on to a carrier.

**Revendications**

1. Agent de diagnostic pour la mise en évidence de composés constitutifs de liquides comprenant un film résistant aux liquides, fabriqué à partir d'une dispersion aqueuse de matières synthétiques organiques filmogènes et à laquelle sont mélangés les réactifs nécessaires à la mise en évidence ainsi que des agents de charge sous la forme de particules minérales et organiques insolubles dont la taille est comprise entre 0,2 et 20 $\mu$m, caractérisé en ce que pour rendre le film accessible aux composés constitutifs dont le poids moléculaire est supérieur à 50.000 ainsi qu'aux composés constitutifs se trouvant sous la forme d'ingrédients corpusculaires, le rapport en poids entre la quantité d'agents de charge et la quantité de matières synthétiques est compris entre 2/1 et 20/1.

2. Agent de diagnostic selon la revendication 1 caractérisé en ce que le film est fixé sur un support.